# EUROPÄISCHE PATENTSCHRIFT

(11) **EP 0 973 543 B1**
(45) Veröffentlichungstag und Bekanntmachung des Hinweises auf die Patenterteilung: **04.02.2004**
(21) Anmeldenummer: 98912140.5
(22) Anmeldetag: 06.04.1998
(51) Int. Cl.: A61K 38/48, C12N 9/74

(54) **VERFAHREN ZUR INAKTIVIERUNG VON PATHOGENEN, INSBESONDERE VON VIREN, IN EINEM BIOLOGISCHEN MATERIAL**
METHOD FOR INACTIVATING PATHOGENS, ESPECIALLY VIRUSES, IN A BIOLOGICAL MATERIAL
PROCEDE DE NEUTRALISATION D'AGENTS PATHOGENES, NOTAMMENT DE VIRUS, DANS UN MATERIAU BIOLOGIQUE

(30) Priorität: 08.04.1997 AT 59497
(43) Veröffentlichungstag der Anmeldung: 26.01.2000
(73) Patentinhaber: Baxter Aktiengesellschaft, 1221 Wien (AT)
(72) Erfinder: SCHWARZ, Hans-Peter, A-1180 Wien (AT); ZERLAUTH, Gerold, A-1090 Wien (AT); TURECEK, Peter, A-3400 Klosterneuburg (AT)
(74) Vertreter: Alge, Daniel, Mag. Dr. rer.nat.
(86) Internationale Anmeldenummer: PCT/AT1998/000090
(87) Internationale Veröffentlichungsnummer: WO 1998/044941

(56) Entgegenhaltungen:
- EP-A- 0 124 506
- EP-A- 0 197 554
- EP-A- 0 541 507
- EP-A- 0 765 669
- GB-A- 2 080 312

## Beschreibung

Die vorliegende Erfindung betrifft ein Verfahren zur Inaktivierung von Pathogenen in einem biologischen Material durch Inkubieren mit einem chemischen Mittel.

Ein biologisches Material stammt von Organismen bzw. Körperflüssigkeiten oder Mikroorganismen.

Da biologisches Material mit Pathogenen, wie z.B. infektiöse Moleküle oder Mikroorganismen und Viren, bzw. Pyrogenen, kontaminiert sein kann, wurden bereits verschiedene Verfahren zur Inaktivierung bzw. Abreicherung von Pathogenen bzw. Pyrogenen entwickelt.

Derartige Verfahren beinhalten physikalische und/oder chemische Behandlungen, wie beispielsweise diverse Filtrationsmethoden (z.B. Nano-, Dia- oder Ultrafiltration), Hitzebehandlung, Behandlung mit Säure oder Lauge, Behandlung mit Detergens und/oder organisches Lösungsmittel sowie Behandlung mit UV-Licht oder mit Laser-Licht. Auch verschiedene Kombinationen solcher Verfahren zur Inaktivierung bzw. Abreicherung von Pathogenen wurden im Stand der Technik vielfach vorgeschlagen.

Aus der EP 0 197 554 ist beispielsweise ein Verfahren zum Depyrogenisieren und Inaktivieren von Viren in einem biologischen oder pharmazeutischen Produkt bekannt, welches eine Behandlung mit einem virusinaktivierenden und depyrogenisierenden Mittel, wie z.B. eine amphiphile Substanz und/oder ein Lösungsmittel, an einer festen Phase, an der das Produkt adsorbiert vorliegt, umfaßt. Nach dieser Behandlung wird das virusinaktivierende und depyrogenisierende Mittel von der festen Phase abgetrennt, das adsorbierte Produkt gewaschen und schließlich von der festen Phase eluiert.

Aus der EP 0 131 740 ist die Behandlung einer Protein enthaltenden Zusammensetzung in einer Lösung mit organischen Lösungsmitteln wie Di- oder Trialkylphosphaten, gegebenenfalls in Gegenwart eines Detergens (Solvens/Detergens-Behandlung) bekannt, wodurch Protein-Zusammensetzungen frei von lipidhältigen Viren erhalten werden können.

Aus der AT-PS 402 151 ist eine Hitzebehandlung bekannt, bei welcher einem in wäßriger Lösung vorliegenden Präparat vor dem Erhitzen ein Tensid in einer Konzentration von mindestens 1 Gew.% zugesetzt wird.

Ein weiteres Verfahren zur Reduktion bzw. Suppression unerwünschter Aktivitäten in biologischen oder pharmazeutischen Produkten ist aus der EP 0 083 999 bekannt. Dieses basiert auf einem verlängerten Kontakt mit einer Lösung oder Suspension eines nicht-denaturierend wirkenden Amphiphils. Das depyrogenisierte Produkt wird zur Entfernung des Amphiphils mit einem Ionenaustauscher behandelt.

Ein Nachteil vieler dieser.aus dem Stand der Technik bekannten Verfahren ist das häufige Auftreten von Aktivitätsverlusten der in den zu behandelnden Zusammensetzungen enthaltenen labilen Proteine, beispielsweise von Blutproteinen. Insbesondere bei Durchführung eines chromatographischen Reinigungsschrittes erfolgt eine Inaktivierung von Proteinen in einem relativ hohen Ausmaß. Ein Abbau von Proteinen kann auch zu einer Aktivierung führen. So ist beispielsweise bekannt, daß der Faktor VII bei einer chromatographischen Reinigung aufgrund autokatalytischer Vorgänge sehr leicht zu unerwünschtem, weil sehr labilen, Faktor VIIa aktiviert wird.

Ein weiterer Nachteil liegt in dem hohen zeitlichen und apparativen Aufwand vieler Verfahren, was deren Praktikabilität stark mindert und deshalb die Anwendung im großtechnischen Maßstab häufig ungeeignet macht.

Der vorliegenden Erfindung liegt die Aufgabe zugrunde, ein für Proteine, insbesondere für labile Blutproteine, schonendes Verfahren zur wirksamen Inaktivierung von Pathogenen in biologischen Materialien zur Verfügung zu stellen, welches auch leicht in einen großtechnischen Maßstab übertragbar ist und ökonomisch durchgeführt werden kann. Insbesondere soll bei dem Verfahren zur Inaktivierung von Pathogenen ein Abbau und eine mögliche Aktivierung hierfür empfindlicher Proteine weitgehend vermieden werden.

Die vorstehend genannte Aufgabe wird dadurch gelöst, daß ein Verfahren zur Inaktivierung von Pathogenen, insbesondere von Viren, in einem biologischen Material durch Inkubieren mit einem chemischen Mittel zur Verfügung gestellt wird, bei welchem das biologische Material an einen festen Träger adsorbiert wird und die Inkubation mit einem chemischen Mittel in Gegenwart eines eluotropen Salzes, entsprechend einer NaCl-Konzentration von mindestens 200 mM, vorzugsweise mindestens 300 mM, vorgenommen wird, wodurch die Inkubation gleichzeitig mit der Elution oder unmittelbar nach der Elution des biologischen Materials erfolgt.

Die Inaktivierung von Pathogenen in Lösung bietet gegenüber der Behandlung eines Adsorbens einige Vorteile. So ist beispielsweise die Praktikabilität eines derartigen Verfahrens in einem homogenen, einphasigen System höher und die Validierung des Inaktivierungsschrittes besser möglich. Auch scheint die bessere Zugänglichkeit von Pathogenen in einer relativ homogenen Phase die Effizienz des Verfahrensschrittes zu erhöhen.

Das biologische Material enthält vorzugsweise ein humanes Protein und ist insbesondere Plasma oder eine Plasmafraktion oder stammt von einer Zellkultur. Vorzugsweise enthält das biologische Material einen Blutfaktor, wie Faktor XII, XI, VIII, V, von Willebrand-Faktor oder Fibrinogen, insbesondere ein Vitamin-K-abhängiges Protein, wie Faktor II, Faktor VII, Faktor IX, Faktor X, Protein C, Protein S bzw. Protein Z.

Die Proteine können als Einzelfaktoren, vorzugsweise in gereinigter Form, oder in einem komplexen Gemisch vorliegen. In einer ganz besonders bevorzugten Ausführungsform enthält das biologische Material mindestens einen Faktor des Prothrombinkomplexes und ist insbesondere eine Prothrombinkomplex enthaltende Fraktion oder ein Faktor VII enthaltendes Material, beispielsweise wird nach einer Kryopräzipitation von Plasma vom entsprechenden Überstand (Kryoüberstand) ausgegangen.

Die erfindungsgemäße Präparation ist bevorzugterweise eine solche mit FEIB-Aktivität (Factor Eight Inhibitor Bypassing-Activity), also eine Präparation, die zur Behandlung von Faktor VIII-Inhibitor-Patienten geeignet ist.

Das von einer Zellkultur stammende Material ist vorzugsweise ein Material, enthaltend rekombinant hergestellte Blutfaktoren, darunter Faktoren der intrinsischen oder extrinsischen Gerinnung, der Fibrinolyse, der Thrombolyse oder deren Inhibitoren, insbesondere Vitamin-K-abhängige Blutfaktoren. Als Zellen kommen die für die Expression rekombinanter Proteine gängigen Zellen in Frage, bevorzugterweise Säugerzellen, wie beispielsweise Vero-, CHO- oder BHK-Zellen. Die entsprechenden Proteine können direkt aus dem rohen Zellextrakt dem erfindungsgemäßen Verfahren zur Inaktivierung gegebenenfalls vorhandener Pathogene unterworfen werden, es kann sich jedoch auch um eine vorgereinigte Zellfraktion handeln.

Das chemische Mittel ist beispielsweise ein Detergens (Amphiphil, Tensid), welches vorzugsweise in einer Menge von mindestens 1%, mehr bevorzugt mehr als 5%, am meisten bevorzugt mehr als 10% enthalten ist; es können aber auch andere chemische Mittel erfindungsgemäß eingesetzt werden, insbesondere solche, für die z.B. eine viruzide, bakterizide oder depyrogenisierende Wirkung bereits bekannt ist, bzw. Mischungen verschiedenster chemischer Mittel.

Die Auswahl ist jedoch dadurch limitiert, daß die Nativität des biologischen Materials nicht wesentlich beeinträchtigt werden soll. Für eine ökonomische Verfahrensweise wird eine Chemikalie gewählt, die die biologische Aktivität des Materials zu mehr als 50% erhält, bezogen auf die Aktivität vor dem Inkubieren, vorzugsweise mindestens 70%, insbesondere mehr als 85%. Erhalt der biologischen Aktivität bedeutet, daß die im biologischen Material enthaltenen Proteine die ihnen natürlicherweise zugeschriebene Funktion bzw. die verschiedenen Funktionen ausüben können. Diese biologische Aktivität kann dann je nach Art des Proteins ermittelt und angegeben werden, beispielsweise mittels eines standardisierten chromogenen Tests oder der Antigen-Bestimmung.

Gegebenenfalls wird das chemische Mittel nach der Inkubation abgetrennt.

Unter einem Detergens ist allgemein eine synthetische, organische, grenzflächenaktive Substanz zu verstehen.

Bevorzugterweise wird bei dem erfindungsgemäßen Verfahren ein nichtionisches Detergens verwendet. Nichtionische Tenside wie Polyether, insbesondere Alkylphenolpolyglykolether, sind u.a. Produkte der Ethoxylierung von Fettsäuren, Fettsäureamiden, Fettaminen, Fettalkoholen, Aminoxiden,. Fettsäureestern von Polyalkoholen und Zuckerestern.

Ein solches Tensid wirkt auf die Proteine nicht denaturierend und ist bevorzugterweise ausgewählt aus der Gruppe Polysorbat und Triton. Als Polysorbat wird beispielsweise Tween® verwendet.

Wenn Detergentien als chemische Mittel eingesetzt werden, so werden diese gemäß einer bevorzugten Ausführungsform ohne den Zusatz anderer Mittel eingesetzt, insbesondere ohne den Zusatz von toxischen organischen Substanzen oder Lösungsmitteln, wie z.B. TNBP. Dadurch wird ein Kontaminationsrisiko minimiert.

Das biologische Material wird gemäß dem erfindungsgemäßen Verfahren mit einem chemischen Mittel inkubiert. Inkubation bedeutet das In-Kontakt-bringen des biologischen Materials mit einer Lösung, Suspension oder Emulsion eines chemischen Mittels für einen zur Inaktivierung gegebenenfalls vorhandener Pathogene bzw. Pyrogene ausreichend langen Zeitraum bei einer bestimmten Temperatur. Das In-Kontakt-bringen kann beispielsweise einfach durch Stehenlassen des Gemisches für einen definierten Zeitraum erfolgen.

Die Inkubation erfolgt gemäß der vorliegenden Erfindung in Gegenwart eines eluotropen Salzes. Unter "eluotropem Salz" ist im folgenden das Salz in Gemisch mit chemischen Mittel oder das Salz in einer komplexen Zusammensetzung zu verstehen, mit der Eigenschaft, adsorbierte Stoffe aus festen oder mit Flüssigkeit getränkten, auch gelartigen Adsorbentien herauszulösen und/oder zu verdrängen. Bevorzugterweise handelt es sich bei dem eluotropen Salz um ein Desorptionsmittel, wie es bei chromatographischen Verfahren zur Anwendung kommt. Der adsorbierte Stoff ist i.a. in Gegenwart des eluotropen Salzes ausreichend löslich, d.h. es werden vorzugsweise Bedingungen gewählt, die das biologische Material nicht präzipitieren.

Die Art und Konzentration des Salzes bzw. der Zusammensetzung wird im allgemeinen je nach verwendetem Adsorbens gewählt. Die eluierende Wirkung eines Salzes hängt beispielsweise von der Polarität des Lösungsmittels ab, nimmt also z.B. in der Reihenfolge Ethanol - Aceton - Methanol - Wasser zu. Das Adsorbens kann eine feste Phase sein, insbesondere eine für die Ionenaustausch-Chromatographie geeignete Matrix. In der das eluotrope Salz enthaltenden Zusammensetzung können auch weitere Zusätze, beispielsweise weitere Salze, enthalten sein. Vorzugsweise handelt es sich bei der Zusammensetzung um eine wäßrige Zusammensetzung mit einem pH-Wert im Bereich zwischen 6,0 bis 8,0, bevorzugterweise um 7,0.

In einer bevorzugten Ausführungsform wird als eluotropes Salz Natriumchlorid verwendet, aber auch andere Alkali- oder auch Erdalkalisalze, darunter CaCl₂. Als eluotrope Salze können auch sogenannte Chaotrope, wie z.B. Harnstoff, Rhodanide oder Guanidin, eingesetzt werden. Die Konzentration des Salzes ist mindestens ≥ 200 mmol/l, vorzugsweise ≥ 300 mmol/l. Die obere Grenze für die eingesetzte Konzentration hängt insbesondere von der Löslichkeit des jeweiligen Salzes ab und liegt für NaCl beispielsweise um 2 mol/l. Chaotrope Substanzen, wie z.B. Harnstoff, können gegebenenfalls sogar bis zu einer Konzentration von 8 mol/l eingesetzt werden.

Die Inkubation des biologischen Materials mit dem chemischen Mittel erfolgt für einen für die Inaktivierung gegebenenfalls vorhandener Pathogene ausreichend langen Zeitraum, vorzugsweise für einen Zeitraum zwischen 10 Minuten und 10 Stunden, am meisten bevorzugt zwischen 1 Stunde und 5 Stunden. Der benötigte Zeitraum für das erfindungsgemäße Verfahren kann mittels Modellviren wie HIV, Sindbis-, FSME- oder Hepatitis-Viren in einem Vorversuch bestimmt werden.

Auch die Wahl der Temperatur beeinflußt den anzuwendenden Zeitraum. In dem erfindungsgemäßen Verfahren wird bevorzugterweise bei Raumtemperatur, beispielsweise in einem Temperaturbereich zwischen 15 und 45°C, insbesondere zwischen 20 und 30°C, inkubiert.

Beim erfindungsgemäßen Verfahren wird das biologische Material bevorzugt an einem festen Träger adsorbiert, gereinigt und die Inkubation unmittelbar nach Elution des gereinigten Materials vorgenommen. Elution und Inkubation können konsekutiv durchgeführt werden, sie können aber auch gleichzeitig erfolgen.

Gemäß einer weiteren bevorzugten Ausführungsform wird die Inkubation nach einer chromatographischen Reinigung eines biologischen Materials vorgenommen, wobei das Eluat noch weiter prozessiert wurde, beispielsweise durch Zentrifugation, Filtration oder andere physikalische Methoden.

Der feste Träger ist bevorzugterweise ein für die Chromatographie geeignetes Material, insbesondere ein für die Ionenaustausch-Chromatographie, hydrophobe Chromatographie oder die Affinitätschromatographie geeignetes Material. Beispielsweise werden Materialien wie Sepharose®, Superdex, Sephadex®, Spherodex®, Toyopearl®, oder anorganische Materialien, wie Hydroxylapatit, verwendet.

Als Ionenaustauscher können Anionenaustauscher-Materialien, wie beispielsweise DEAE-Sephacel®, DEAE-Sephadex®, DEAE-Sepharose® CL6B, DEAE-Sepharose®Fast Flow, QAE-Sephadex®, Q-Sepharose® Fast Flow, Q-Sepharose® High Performance, DEAE-Tris-Acryl, DEAE-Spherodex®, Q-Hyper-D (erhältlich durch die Firma Sepracor), DEAE-Toyopearl®, QAE-Toyopearl®, Fractogel® EMD-TMAE oder andere Fractogel-Material verwendet werden.

Als Beispiele für hydrophobe Chromatographiematerialien sollen beispielsweise Butyl-Sepharose®, Octyl-Sepharose®, Phenyl-Sepharose®, Fractogel®TSK-Butyl, t-Butyl-HIC Support oder TSK-Gel Butyl Toyopearl® genannt werden.

Das biologische Material kann direkt aus einem komplexen Gemisch an den Träger adsorbiert und gereinigt werden, dem Inaktivierungsschritt können aber auch weitere Schritte zur Reinigung des Materials vor- oder nachgeschaltet werden, wobei im Rahmen der vorliegenden Erfindung weitere chromatographische Reinigungsschritte bevorzugt werden.

Durch das erfindungsgemäße Verfahren werden Pathogene inaktiviert. Unter Pathogenen werden auch Bruchstücke von z.B. Viren, insbesondere auch das isolierte Genom oder dessen Fragmente, verstanden.

Die Pathogene können lipidumhüllte Pathogene, wie z.B. Hepatitis B-Virus oder nicht-lipidumhüllte Pathogene, wie z.B. Hepatitis A-Virus, sein.

Virusinaktivierungsverfahren werden heutzutage als wirksam bezeichnet, wenn nach Anwendung des Verfahrens an einer Probe eines biologischen Materials, welche mit einer hohen Dosis eines Testvirus, z.B. HI-Virus oder Sindbis-Virus als Modellvirus für Hepatitis-Viren, versetzt wurde, keine Viren mehr in der Probe nachgewiesen werden können und der Virustiter somit unter die Nachweisgrenze reduziert wurde. Nachweis und Quantifizierung von Nukleinsäuren kann beispielsweise mittels einer PCR-Methode, wie in der AT-PS 401 062 beschrieben, erfolgen oder durch direkte Titration.

Als Maß für die Inaktivierung ist der sogenannte Reduktionsfaktor bekannt, der nach einer eimaligen Zugabe von Testvirus aus dem dekadischen Logarithmus des Quotienten von Anfangs- und Endvirustiter errechnet wird. Aus der Europäischen Richtlinie EC III/8115/89-EN der Kommission der Europäischen Gemeinschaften ist weiters der sogenannte Gesamt-Reduktionsfaktor bekannt. Er wird aus der Summe der Reduktionsfaktoren von einzelnen subsequenten Inaktivierungsmaßnahmen errechnet.

Auch ein weiterer unabhängiger Schritt zur Inaktivierung bzw.

Abreicherung von Pathogenen wird vorzugsweise durchgeführt. Hierfür kommen alle aus dem Stand der Technik bekannten Verfahren in Frage, um das Infektionsrisiko zu minimieren.

Insbesondere wird als ein weiterer Schritt zur Inaktivierung bzw. Abreicherung eine Filtration und/oder eine Hitzebehandlung vorgenommen.

Als Filtration wird vorzugsweise eine Nanofiltration vorgenommen. Eine bevorzugte Hitzebehandlung wird am festen biologischen Material durchgeführt, z.B. an einem Lyophilisat mit kontrolliertem Wassergehalt, beispielsweise einem Wassergehalt zwischen 5 bis 8% und einer Temperatur zwischen 50 und 80°C, wie in der EP-0 159 311 beschrieben.

In einer bevorzugten Ausführungsform ist eine 2-stufige Behandlung mit einem Detergens als chemischem Mittel vorgesehen. Dabei wird in einem ersten Schritt ein Detergens in einer Menge von wenigstens 1%, vorzugsweise wenigstens 5%, am meisten bevorzugt wenigstens 10% verwendet. In einer zweiten Stufe wird ein weiteres Detergens in einer Menge von mindestens 10%, vorzugsweise mindestens 12%, am meisten bevorzugt mindestens 14% verwendet. Das verwendete Detergens kann für beide Stufen dasselbe sein, es können aber auch unterschiedliche Detergentien eingesetzt werden. Ganz allgemein kann durch die Kombination von Schritten zur Virusinaktivierung das Risiko einer Virusinfektion nach Verabreichung einer entsprechenden Präparation stark reduziert bzw. ausgeschlossen werden.

Gemäß der vorliegenden Erfindung wird auch eine chromatographisch gereinigte Präparation zur Verfügung gestellt, enthaltend einen autodynamisch aktivierbaren Blutfaktor mit einem Anteil an aktiviertem Blutfaktor von weniger als 50%, bezogen auf den Gehalt an dem aktivierten und nicht aktivierten Blutfaktor, vorzugsweise weniger als 40%, mehr bevorzugt weniger als 30%, noch mehr bevorzugt weniger als 20%, weiters bevorzugt weniger als 10%, am meisten bevorzugt weniger als 1%, und einem Detergens-Gehalt.

Insbesondere handelt es sich bei der Präparation um eine einen Prothrombinkomplex enthaltende Präparation mit einer Faktor VIIa-Aktivität von weniger als 50%, bezogen auf den Gehalt an aktiviertem und nicht aktiviertem Faktor VII, vorzugsweise weniger als 10%, am meisten bevorzugt weniger als 1%. Der Detergensgehalt des erfindungsgemäßen Präparates liegt dabei in einer pharmazeutisch akzeptablen Menge vor, vorzugsweise zwischen 1% und der Nachweisgrenze des Detergens.

Unter einem autodynamisch aktivierbaren Blutfaktor ist gemäß der vorliegenden Erfindung ein Blutfaktor zu verstehen, der autokatalytisch, durch Oberflächenkontakt oder durch Prozesse, wie beispielsweise chromatographische Prozesse, aktivierbar ist. Insbesondere ist ein derartiger Blutfaktor ein solcher, ausgewählt aus der Gruppe Faktor VII, Faktor XII, Faktor XI und Prä-Kallikrein.

In einer weiteren bevorzugten Ausführungsform ist die Präparation frei von Serinprotease-Inhibitoren, wie beispielsweise Thrombin-Inhibitoren, bzw. der Kofaktoren, wie z.B. Heparin. In einer speziellen Ausführungsform ist die Freiheit von derartigen Substanzen bereits während eines chromatographischen Prozesses gegeben.

Daher betrifft die vorliegende Erfindung, auch entsprechende Präparationen, erhältlich durch das erfindungsgemäße Verfahren.

In der erfindungsgemäßen Präparation können auch weitere Zusätze enthalten sein, beispielsweise stabilisierend wirkende Substanzen wie Aminosäuren.

Durch die nachfolgenden Beispiele soll die vorliegende Erfindung noch näher erläutert werden, ohne diese jedoch darauf zu beschränken.

### BEISPIEL 1 :

### Detergensbehandlung von aktiviertem Prothrombinkomplex FEIBA in Gegenwart von TWEEN®-80

15 mg DEAE-Sephadex® A-50, Fa. Pharmacia, wurden 15 min bei Raumtemperatur mit 1 ml einer Lösung von 30 g/l NaCl in Wasser zur Quellung inkubiert. Danach würde das Gel durch Zentrifugation vom Quellüberstand abgetrennt. Anschließend folgten fünf Waschungen des Gels mit je 1 ml Puffer (9 g/l Na₂HPO₄.2H₂O, 7 g/l NaCl, pH 7,0) und weitere zwei Waschungen mit einem Puffer (7 g/l Na₃ Citrat.2H₂O, 7 g/l NaCl) ebenfalls durch Resuspendieren und Zentrifugieren.

30 ml frisch gefrorenes menschliches Citratplasma wurden bei 0 bis +4°C aufgetaut und das anfallende Kryopräzipitat durch Zentrifugation bei +2°C abgetrennt. Der daraus resultierende "Kryoüberstand" wurde mit dem gewaschenen DEAE-Sephadex® inkubiert, wobei FEIBA generiert und zusammen mit den Faktoren des Prothrombinkomplexes und inertem Protein an das Gel adsorbiert wurde. Danach wurde coadsorbiertes Inertprotein vom DEAE-Gel durch Waschen mit einem Puffer (9 g/l Na₂HPO₄.2H₂O, 7 g/l NaCl) entfernt.

Der pufferfeuchte Gel-Proteinkomplex wurde nun mit 1,5 ml einer Lösung von 150 mg/ml TWEEN®-80 und 30 mg/ml NaCl 1 Stunde bei 26°C suspendiert. Durch die Behandlung mit der Lösung hoher Ionenstärke wurde Protein gemeinsam mit den Faktoren des Prothrombinkomplexes und eventuell vorhandenen Pathogenen desorbiert. Anschließend wurde die Suspension durch Zugabe von 6,5 ml Wasser verdünnt und 1 Stunde bei Raumtemperatur readsorbiert, wobei die Proteinfraktion neuerlich readsorbiert wurde, während Bestandteile des inaktivierten Pathogens gemeinsam mit dem Detergens in Lösung blieben. Der Gel/Proteinkomplex wurde dann fünf mal mit je 1 ml einer Lösung von 7 g/l NaCl in Wasser detergensfrei gewaschen.

Zur Elution wurde das Gel mit 0,7 ml einer Lösung von 30 g/l NaCl in Wasser unter Rühren behandelt. Das Eluat wurde nun gegen destilliertes Wasser dialysiert, eingefroren und lyophilisiert. Nach Rekonstitution des Lyophilisates wurde die FEIB-Aktivität gemäß der AT-B 350 726 bestimmt.

Als Kontrolle dienten eine ebenso hergestellte Präparation von FEIBA, jedoch ohne Behandlung mit Detergens.

Die Analyse des gewonnenen Präparates zeigte eine spezifische Aktivität von 3,2 E FEIBA/mg Protein bei einem Proteingehalt von 16,6 mg/ml nach Rekonstitution des Lyophilisates und war vergleichbar mit der Verfahrensvariante ohne Detergensbehandlung, wobei eine spezifische Aktivität von 2,8 E/mg Protein bei einer Proteinkonzentration von 16,5 mg/ml erhalten wurde.

### BEISPIEL 2 :

### Detergensbehandlung bei der Desorption von FEIBA mit verlängerter Inkubationszeit

Analog zu Beispiel 1 wurde die Prothrombinkomplexfraktion an DEAE-Sephadex® adsorbiert, von Inertprotein freigewaschen, anschließend mit einer TWEEN®/NaCl-Lösung desorbiert. Die Proteinfraktion wurde aber über 2 bzw. 3 Stunden im desorbierten Zustand unter sonst gleichbleibenden Bedingungen gehalten. Anschließend wurde, wie in Beispiel 1 beschrieben, zum Endprodukt aufgearbeitet .

Die Analyse diese Ansätze ergab eine spezifische Aktivität von 2,5 E FEIBA/mg Protein bei einem Proteingehalt von 16,6 mg/ml bei 2 h Inkubation in Gegenwart von TWEEN®-80 und eine spezifische Aktivität von 2,3 E FEIBA/mg Protein bei einem Proteingehalt von 17,4 mg/ml bei 3 h Inkubation mit Detergens.

Damit konnte gezeigt werden, daß auch die verlängerte Kontaktzeit mit dem Detergens mit keiner wesentlichen Inaktivierung des Wirkstoffes oder Reduktion der Ausbeute verbunden war.

### BEISPIEL 3 :

### Detergensbehandlung von FEIBA mit Readsorption an einem anderen Gel

FEIBA wurde wie in Beispiel 1 beschrieben hergestellt. Nach der Behandlung und Desorption mit Detergens wurde die erhaltene Lösung in einen Behälter übergeführt in welchem 15 mg DEAE-Sephadex® A50, Fa. Pharmacia, welcher in einer Lösung von 30 g/l NaCl zur Quellung vorinkubiert und anschließend durch fünf Waschungen mit je 1 ml eines Puffers (9 g/l Na₂HPO₄ .2H₂O, 7 g/l NaCl, pH 7,0) und weitere zwei Waschungen mit einem Puffer (7 g/l Na₃Citrat.2H₂O, 7g/l NaCl) jeweils durch Resuspendieren und

Zentrifugieren vorgelegt worden war. Nach einstündiger Adsorption des verdünnten Proteinkomplexes zur Abtrennung des Detergens wurde die Aufarbeitung nach dem in Beispiel 1 beschriebenen Verfahren durchgeführt. Das so gewonnene Endprodukt hatte im Vergleich zu einer nach der Standardvariante, d.h. ohne Behändlung mit Detergens, hergestellten FEIBA eine Ausbeute von 95 % und war in der spezifischen Aktivität vergleichbar.

### BEISPIEL 4 :

### Detergensbehandlung von aktiviertem Prothrombinkomplex FEIBA in Gegenwart von TWEEN®-80 bei erhöhter Temperatur

15 mg DEAE-Sephadex® A-50, Fa. Pharmacia, wurden 15 min bei Raumtemperatur mit 1 ml einer Lösung von 30 g/l NaCl in Wasser zur Quellung inkubiert. Danach wurde das Gel durch Zentrifugation vom Quellüberstand abgetrennt . Anschließend folgten fünf Waschungen des Gels mit je 1 ml Puffer (9 g/l Na₂HPO₄.2H₂O, 7 g/l NaCl, pH 7,0) und weitere zwei Waschungen mit einem Puffer (7 g/l Na₃Citrat.2H₂O, 7 g/l NaCl) ebenfalls durch Resuspendieren und Zentrifugieren.

30 ml frisch gefrorenes menschliches Citratplasma wurden bei 0 bis +4°C aufgetaut und das anfallende Kryopräzipitat durch Zentrifugation bei +2°C abgetrennt. Der daraus resultierende "Kryo-überstand" wurde mit dem gewaschenen DEAE-Sephadex® inkubiert, wobei FEIBA generiert und zusammen mit den Faktoren des Prothrombinkomplexes und inertem Protein an das Gel adsorbiert wurde. Danach wurde coadsorbiertes Inertprotein vom DEAE-Gel durch Waschen mit einem Puffer (9 g/l Na₂HPO₄ .2H₂O, 7 g/l NaCl) entfernt.

Der pufferfeuchte Gel-Proteinkomplex wurde nun mit 1,5 ml einer Lösung von 1 mg/ml TWEEN®-80 und 30 mg/ml NaCl 1 Stunde bei Raumtemperatur suspendiert, wobei die Proteinfraktion und unspezifisch adsorbierte Verunreinigungen desorbiert wurden. Anschließend wurde das Gel durch Filtration abgetrennt. Die Proteinlösung wurde nun durch weiteren Zusatz von TWEEN®-80 auf eine Detergenskonzentration von 150 mg/ml gebracht und anschließend entweder 1 Stunde bei 26°C oder 1 Stunde bei 40°C unter Rühren zur Inaktivierung eventuell vorhandener Pathogene inkubiert. Anschließend wurde durch die Zugabe von 6,5 ml Wasser verdünnt und ein frisch gewaschenes vorbereitetes DEAE-Sephadex® A-50 Gel readsorbiert. Anschließend wurde durch fünf Waschungen mit je 1 ml einer Lösung von 7 g/l NaCl in Wasser detergensfrei gewaschen und schließlich das Präparat, wie in Beispiel 1 beschrieben, weiteraufgearbeitet.

Die Analyse beider Varianten der Behandlung bei 26°C und bei 40°C zeigte eine mit einer Standardvariante ohne Virusinaktivierung vergleichbare spezifische Aktivität des FEIBA-Präparates. Die Ausbeuten betrugen jeweils 75 % der Standardvariante.

### BEISPIEL 5 :

### Detergensbehandlung von Prothrombinkomplex in Gegenwart von TWEEN®-80 (Derzeit nach Ansicht der Anmelderin der beste Weg zur Ausführung der Erfindung)

30 ml frisch gefrorenes menschliches Citratplasma wurden bei 0 bis +4°C aufgetaut und das dabei anfallende Kryopräzipitat durch Zentrifugation bei +2°C abgetrennt. Der daraus resultierende "Kryoüberstand" wurde mit 2 IE Heparin/ml versetzt. Danach wurden die Proteine des Prothrombinkomplexes mit DEAE-Sephadex® A-50, Fa. Pharmacia, in einer Konzentration von 0,5 mg/ml adsorbiert. Der Gel-Proteinkomplex wurde von der Lösung abgetrennt und jeweils mit einem Puffer 1 (4 g/l Na₃Citrat.2H₂O, 7 g/l NaCl, 9 g/l Na₂HPO₄ .2H₂O, 500 IE Heparin/l, pH 7,5) und anschließend mit Puffer 2 (4 g/l Na₃Citrat.2H₂O/l, 7 g/l NaCl, 500 IE Heparin/l, pH 7,5) gewaschen.

Das gewaschene Gel wurde nun zur Pathogeninaktivierung mit 1,5 ml einer Lösung, enthaltend 150 mg TWEEN®-80/ml und 30 mg NaCl/ml, 1 h bei 26°C suspendiert. Durch diese Behandlung wurde die Proteinfraktion gemeinsam mit eventuell vorhandenen Pathogenen oder Pathogenfragment desorbiert und im Laufe der Inkubation mit dem Detergens wurde Pathogen inaktiviert. Anschließend wurde wie im Beispiel 1 beschrieben mit 6 ml Wasser verdünnt und 1 h bei Raumtemperatur die Proteinfraktion samt Wirkstoff an die Ionenaustauschermatrix readsorbiert. Anschließend wurde fünf mal mit 1 ml eines Puffers (4 g/l Na₃Citrat, 7 g/l NaCl, 500 IE Heparin/l, pH 7,5) detergensfrei gewaschen und mit einer Lösung von 1 g/l Na₃ Citrat.2H₂O, 30 g/l NaCl, 1000 IE Heparin, pH 7,0 eluiert. Zum Eluat wurde 1 IE Heparin/ml zugesetzt. Die Prothrombinkomplex enthaltende Lösung wurde gegen einen Puffer enthaltend 4 g/l Na₃Citrat.2H₂O, 8 g/l NaCl, pH 7,0 umgepuffert und lyophilisiert. Im rekonstituierten lyophilisierten Prothrombinkomplex wurden der Proteingehalt und der Gehalt an Prothrombinkomplexfaktoren getestet; die Resultate sind Tabelle 1 zu entnehmen.

Als Kontrolle wurde ein Ansatz ohne TWEEN®-Behandlung hergestellt. Die Analysenergebnisse sind ebenso Tabelle 1 zu entnehmen .

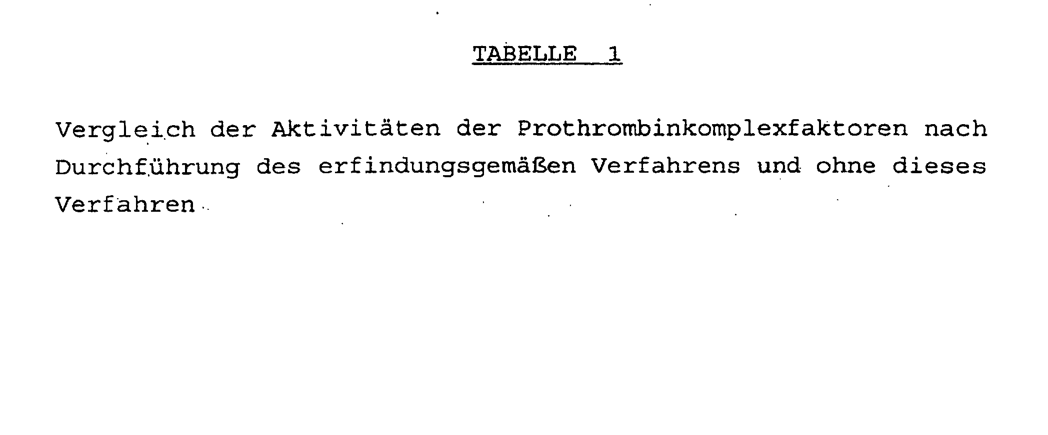

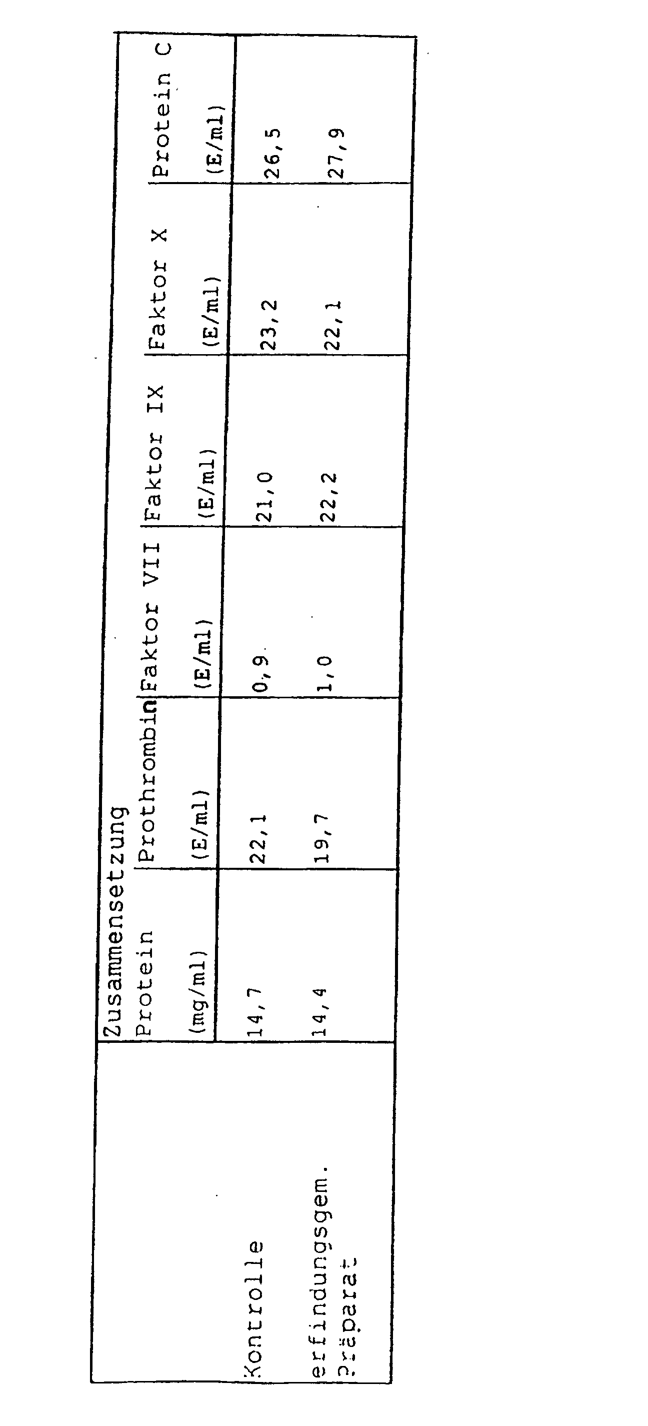

Es zeigte sich, daß durch die Detergensbehandlung keine wesentliche Änderung der Zusammensetzung des Prothrombinkomplexes erfolgte.

### BEISPIEL 6 :

### Detergensbehandlung von Faktor VII mit TWEEN®-80 im Vergleich zur Virusinaktivierung von Faktor VII nach einem konventionellen Verfahren

Aus humanem Citratplasma wurde die Prothrombinkomplexfraktion enthaltend die Gerinnungsfaktoren Prothrombin, geringe Teile von Faktor VII, Faktor IX und Faktor X, wie in Beispiel 5 beschrieben, abgetrennt. Der im Überstand nach Adsorption an DEAE-Sephadex® A50 verbleibende Großteil des Gerinnungsfaktors VII wurde nun durch Adsorption an Aluminiumhydroxid gewonnen. Dazu wurden pro 1 1 Überstand nach Abtrennung des Prothrombinkomplexes 10 ml 2 %iger Aluminium-Hydrogelsuspension zugesetzt und 30 min bei 4°C gerührt. Anschließend wurde durch Zentrifugation bei 5000 rpm 10 min bei ca. 4°C in einem Sorvall RC3B Rotor H6000A der Aluminiumhydroxid-Proteinkomplex abgetrennt, der Überstand verworfen und der Niederschlag mit 3,5 % des Volumens des zur Adsorption verwendeten Prothrombinkomplexüberstandes in einer Lösung von 4 g Na₃Citrat.2H₂O/l und 7 g NaCl/l, pH 7,5, suspendiert und 30 min gerührt. Dadurch wurde Inertprotein vom Aluminiumhydroxid desorbiert. Der am Aluminiumhydroxid verbleibende Faktor VII wurde durch neuerliche Zentrifugation wie oben beschrieben pelletiert. Der Überstand wurde verworfen und der Niederschlag zur Weiterverarbeitung weiterverwendet. Zur Desorption der Proteinfraktion wurde der Aluminiumhydroxid-Faktor VII-Komplex mit 1 Vol.% des zur Adsorption verwendeten Prothrombinkomplexüberstandes eines 0,3 mol/l Phosphatpuffers, pH 8,6, (53,4 g Na₂HPO₄ .2H₂ O/l wurden mit einer Lösung von 41,1 g NaH₂PO₄H₂O/l auf pH 8,6 eingestellt) enthaltend 1 % TWEEN®-80, 30 min gerührt. Anschließend wurde zur Pathogeninaktivierung auf eine Endkonzentration von 15 % TWEEN®-80 Detergens zugesetzt und anschließend 1 Stunde bei 40°C gerührt. Danach wurde die Lösung auf ca. 22°C abgekühlt und mit 9 Teilen Aqua dest. verdünnt. Die Faktor VII-Fraktion wurde dann an 1 g/l DEAE-Sephadex® A50 unter einstündigem Rühren bei ca. 22°C readsorbiert. Anschließend wurde auf der Sinternutsche der Gel-Proteinkomplex durch dreimaliges Waschen mit je 100 ml pro Liter eingesetzter, verdünnter TWEEN®-Lösung mit einem Puffer enthaltend 4 g/l Na₃Citrat.2H₂O/l und 7 g NaCl/l, pH 7,5, enthaltend 500 IE Heparin/l, vom Detergens freigewaschen. Die Elution der Faktor VII-Fraktion erfolgte durch Rühren des Ionenaustauscherproteinkomplexes und 100 ml/l verdünnter TWEEN®-Lösung einer 85 g NaCl/l enthaltenden Lösung für 30 min bei 22°C. Im Eluat wurde anschließend der Faktor VII-Gehalt mit einem chromogenen Faktor VII-Test (Immunochrom Faktor VII:C, IMMUNO AG, Wien, gemessen gegen den internationalen Prothrombinkomplex-Standard), der Proteingehalt nach der Methode von Bradford [Anal.Biochem. 72:248-254 (1976)] und Faktor VIIa, nach der Methode aus US683,682 (gemessen gegen den internationalen Faktor VIIa-Standard) quantitativ bestimmt. Die Ergebnisse sind Tabelle 2 zu entnehmen.

Zum Vergleich wurde Faktor VII durch Adsorption an Aluminiumhydroxid wie oben beschrieben von den anderen Proteinen des Prothrombinkomplexes abgetrennt und im adsorbierten Zustand gemäß der EP 0 197 554 mit den virusinaktivierenden Agentien aus EP 0 131 740 mit TWEEN®-80 und Tri-(N-butyl)phosphat (TNBP) behandelt. Dazu wurde der Alhydrogel-Proteinkomplex in einer wäßrigen Lösung von 1 % TWEEN®-80 und 0,3 % Tri-(N-butyl)phosphat 18 Stunden bei 4°C mit einem Volumen von 50 ml/l Prothrombinkomplexüberstand gerührt. Anschließend wurde wie oben beschrieben zur Abtrennung des Aluminiumhydroxid-Proteinkomplexes zentrifugiert und durch Waschung mit 3 x 100 ml einer Lösung von 4 g/l Na₃ Citrat.2H₂O, 7 g/l NaCl, pH 7,5, durch Resuspendieren von überschüssigem TWEEN®-80 und Tri-(N-butyl)phosphat befreit. Zwischen jeder Waschung folgte eine Pelletierung des Aluminiumhydroxid-Proteinkomplexes durch Zentrifugation. Die Elution wurde unter den gleichen Bedingungen wie beim Parallelansatz nach dem erfindungsgemäßen Verfahren durchgeführt. Ebenso wurden die Analysen des Endproduktes analog durchgeführt. Die Resultate sind Tabelle 2 zu entnehmen.

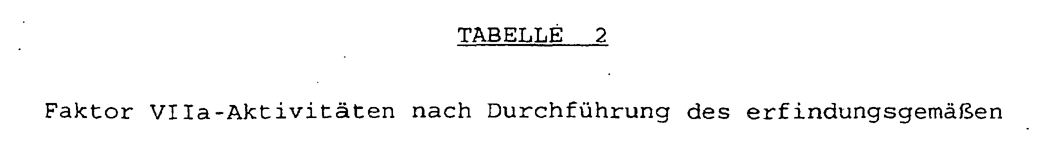

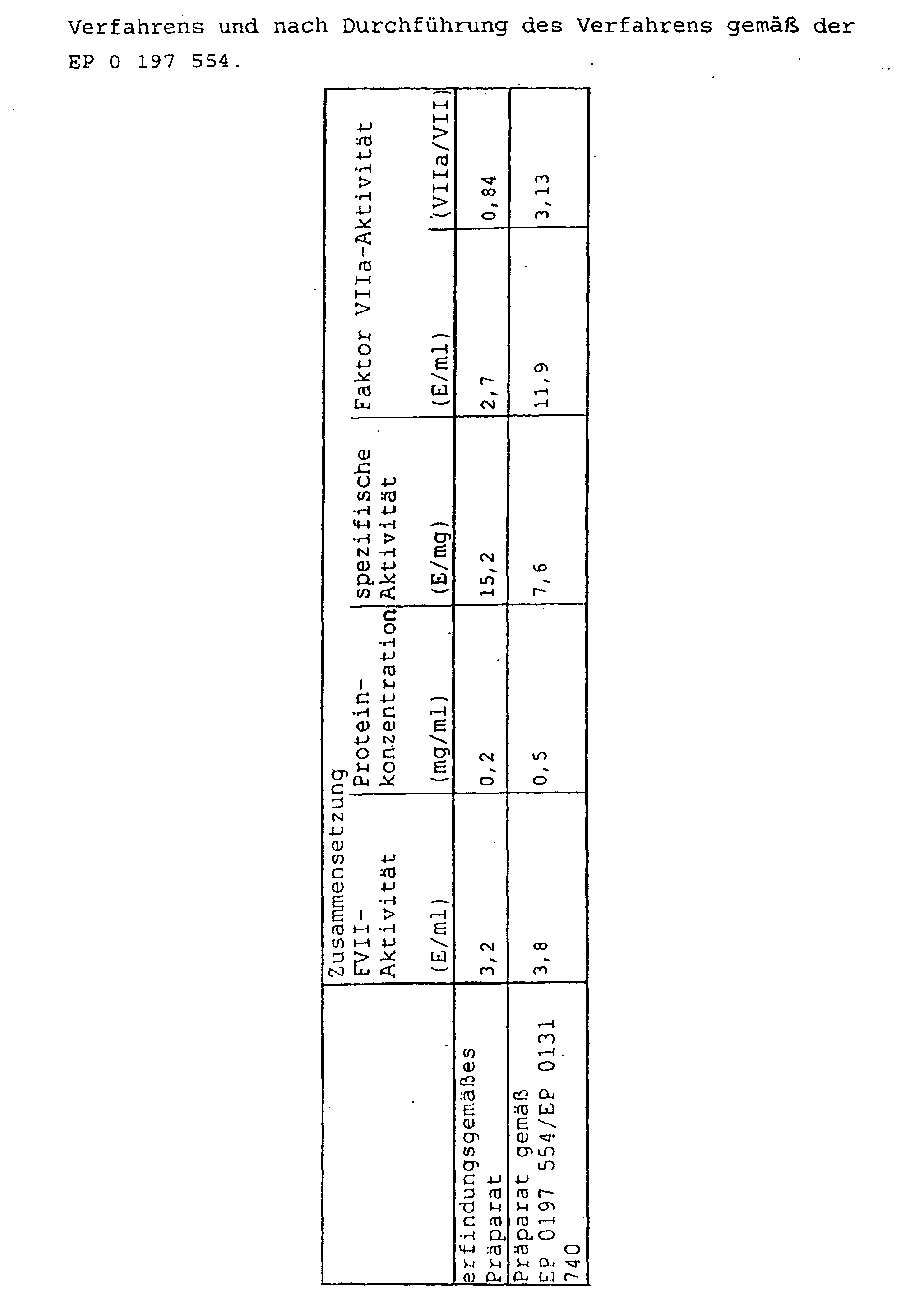

Es zeigte sich, daß unter Anwendung dieses Verfahrens der Faktor VIIa-Gehalt im Vergleich zum erfindungsgemäßen Verfahren deutlich erhöht war, wobei trotz der komplexen Behandlung des Faktor VII, keine Aktivierung festzustellen war. Außerdem war bei dem erfindungsgemäßen Verfahren die spezifische Aktivität des erhaltenen Produktes höher als beim Vergleichspräparat.

### BEISPIEL 7 :

### Semiquantitative Bestimmung von Hepatitis G-Virus

In den Pathogeninaktivierungsansätzen der Beispiele 1 bis 6 wurden Proben jeweils von den Ausgangsmaterialien, Überstand nach Kryopräzipitation oder Adsorptionsüberstand nach Abtrennung der Gerinnungsfaktoren II, IX und X, sowie den entsprechenden gereinigten und konzentrierten Gerinnungsfaktorpräparaten gezogen. 0,5 ml dieser Proben wurden 1 + 1 mit physiologischem Phosphat-Kochsalzpuffer verdünnt und eventuell vorhandene Viren wurden durch Ultrazentrifugation pelletiert. Die RNA wurde aus den viralen Pellets durch die RNAzol-Reagens-Methode (Biotecx, Houston, Texas) extrahiert und in sterilem A.dest. gelöst.

RT-PCR auf Hepatitis G-Virus (HGV)-Nukleinsäuren wurde mit dem Primerpaar NS5a 1 und NS5a 2 (Linnen, J. et al., Science 271: 505-508 (1996)), durchgeführt. Die Sequenz der verwendeten Primer (erhältlich von Boehringer Mannheim, Deutschland) war für NS5a 1: 5'CTCTTTGTGGTAGTAGCCGAGAGAT 3' und für NS5a 2: 5'CGAATGAGTCAGAGGACGGGGTAT 3'. Die Primer wurden mit einem Fluoreszenzfarbstoff markiert und die daraus nach Routineverfahren üblicher PCR-Protokolle resultierenden fluoreszierenden Amplicons wurden auf einem ABI 377-Sequencer von Applied Biosystems analysiert. Um die Anwesenheit von RT-PCR-Inhibitoren in den Proben ausschließen zu können, wurden die Proben mit Hepatitis C-Virus-RNA-Mimics gespikt und in einer, gemäß EP 0 714 988, durchgeführten Hepatitis C-PCR analysiert. Es wurden ausschließlich Extrakte, die keine Inhibition in der HCV-PCR zeigten, als für die HGV-PCR auswertbar angenommen. Die Intensität der Fluoreszenz wurde als Maß für den Gehalt an Hepatitis G-Virus genommen. Es zeigte sich, daß die für die Fraktionierung verwendeten Ausgangsmaterialien vor der Pathogeninaktivierung gemäß dem erfinderischen Verfahren stark positive Signale, d.h. eine hohe Konzentration an HGV-Nukleinsäureamplifikaten, aufwiesen, während in den Eluaten nach der Readsorption und Abtrennung der virusinaktivierenden Agentien keine HGV-RNA mehr nachweisbar war.

Bei Parallelversuchen ohne Durchführung einer Detergensbehandlung waren die Eluate wie die verwendeten Ausgangsmaterialien HGV-PCR-positiv.

## Patentansprüche

1. Verfahren zur Inaktivierung von Pathogenen, insbesondere von Viren in einem biologischen Material durch Inkubieren mit einem chemischen Mittel, **dadurch gekennzeichnet, dass** das biologische Material an einen festen Träger adsorbiert wird und danach die Inkubation mit einem chemischen Mittel in Gegenwart eines eluotropen Salzes, entsprechend einer NaCl-Konzentration von mindestens 200 mM, vorzugsweise mindestens 300 mM, zur Herauslösung und/oder Verdrängung der adsorbierten Stoffe aus dem Träger vorgenommen wird, wodurch die Inkubation gleichzeitig mit der Elution oder unmittelbar nach der Elution des biologischen Materials erfolgt.

2. Verfahren nach Anspruch 1, **dadurch gekennzeichnet, daß** als chemische Mittel ein Detergens verwendet wird, welches vorzugsweise in einer Menge von mindestens 1 %, mehr bevorzugt mehr als 5 %, am meisten bevorzugt mehr als 10 % enthalten ist.

3. Verfahren nach Anspruch 1 oder 2, **dadurch gekennzeichnet, daß** als eluotropes Salz Natriumchlorid verwendet wird.

4. Verfahren nach einem der Ansprüche 1 bis 3, **dadurch gekennzeichnet, daß** die Inkubation für einen Zeitraum zwischen 10 Minuten und 10 Stunden, am meisten bevorzugt zwischen 1 Stunde und 5 Stunden, erfolgt.

5. Verfahren nach einem der Ansprüche 1 bis 4, **dadurch gekennzeichnet, daß** als biologisches Material Plasma oder eine Plasmafraktion verwendet wird oder Material von einer Zellkultur.

6. Verfahren nach einem der Ansprüche 1 bis 5, **dadurch gekennzeichnet, daß** ein biologische Material verwendet wird, das einen Blutfaktor, insbesondere ein Vitamin-K-abhängiges Protein, enthält.

7. Verfahren nach einem der Ansprüche 1 bis 6, **dadurch gekennzeichnet, daß** ein biologisches Material verwendet wird, das eine Prothrombinkomplex enthaltende Fraktion ist.

8. Verfahren nach einem der Ansprüche 1 bis 7, **dadurch gekennzeichnet, daß** das biologische Material an einem festen Träger adsorbiert, gereinigt und die Inkubation nach Elution des gereinigten Materials vorgenommen wird.

9. Verfahren nach Anspruch 8, **dadurch gekennzeichnet, daß** die Elution und die Inkubation gleichzeitig erfolgen.

10. Verfahren nach Anspruch 8 oder 9, **dadurch gekennzeichnet, daß** als fester Träger ein chromatographisches Material verwendet wird, insbesondere ein für die Ionenaustausch-Chromatographie oder die Affinitätschromatographie geeignetes Material.

11. Verfahren nach einem der Ansprüche 1 bis 10, **dadurch gekennzeichnet, daß** weitere Schritte zur Reinigung des Materials durchgeführt werden, insbesondere eine chromatographische Reinigung.

12. Verfahren nach einem der Ansprüche 1 bis 11, **dadurch gekennzeichnet, daß** ein weiterer Schritt zur Inaktivierung bzw. Abreicherung von Pathogenen durchgeführt wird, insbesondere eine Filtration oder eine Hitzebehandlung.

13. Verfahren nach einem der Ansprüche 1 bis 12, **dadurch gekennzeichnet, daß** als chemisches Mittel ein nicht-ionisches Detergens ausgewählt aus der Gruppe Tween und Triton verwendet wird.

14. Chromatographisch gereinigte Präparation, enthaltend einen autodynamisch aktivierbaren Blutfaktor mit einem Anteil von weniger als 50% an aktiviertem Blutfaktor, bezogen auf den Gehalt an dem aktivierten und nicht aktivierten Blutfaktor, vorzugsweise weniger als 40%, mehr bevorzugt weniger als 30%, noch mehr bevorzugt weniger als 20%, weiters bevorzugt weniger als 10%, am meisten bevorzugt weniger als 1%, und einem Detergens-Gehalt.

15. Präparation nach Anspruch 14, **dadurch gekennzeichnet, daß** der Blutfaktor ausgewählt ist aus der Gruppe Faktor VII, Faktor XII, Faktor XI und Prä-Kallikrein.

16. Präparation nach einem der Ansprüche 14 oder 15, **dadurch gekennzeichnet, daß** sie einen Prothrombinkomplex enthält mit einer Faktor VIIa-Aktivität von weniger als 50%, bezogen auf den Gehalt an aktiviertem und nicht aktiviertem Faktor VII, vorzugsweise weniger als 10%, am meisten bevorzugt weniger als 1%.

17. Präparation nach einem der Ansprüche 14 bis 15, **dadurch gekennzeichnet, daß** die Präparation frei ist von Serinprotease-Inhibitoren bzw. deren Kofaktoren.

18. Präparation nach einem der Ansprüche 14 bis 17, erhältlich durch ein Verfahren nach einem der Ansprüche 1 bis 13.

## Claims

1. A method for inactivating pathogens, in particular viruses, in a biological material by incubation with a chemical agent, **characterised in that** the biological material is adsorbed on a solid carrier and subsequently the incubation is carried out with a chemical agent in the presence of an eluotropic salt corresponding to a NaCl concentration of at least 200 mM, preferably at least 300 mM, to dissolve out and/or displace the adsorbed substances from the carrier, whereby the incubation is effected simultaneously with the elution or immediately after the elution of the biological material.

2. A method according to claim 1, **characterised in that** a detergent is used as the chemical agent, which is preferably contained in an amount of at least 1%, more preferred more than 5%, most preferred more than 10%.

3. A method according to claim 1 or 2, **characterised in that** sodium chloride is used as the eluotropic salt.

4. A method according to any one of claims 1 to 3, **characterised in that** the incubation is carried out for a period of time of between 10 min and 10 h, most preferred between 1 h and 5 h.

5. A method according to any one of claims 1 to 4, **characterised in that** plasma or a plasma fraction or material from a cell culture is used as the biological material.

6. A method according to any one of claims 1 to 5, **characterised in that** a biological material is used which comprises a blood factor, in particular a vitamin K-dependent protein.

7. A method according to any one of claims 1 to 6, **characterised in that** a biological material is used which is a prothrombin complex-containing fraction.

8. A method according to any one of claims 1 to 7, **characterised in that** the biological material is adsorbed on a solid carrier, is purified, and incubation is carried out after elution of the purified material.

9. A method according to claim 8, **characterised in that** elution and incubation are effected simultaneously.

10. A method according to claim 8 or 9, **characterised in that** a chromatographic material is used as the solid carrier, in particular a material suitable for ion exchange chromatography or affinity chromatography.

11. A method according to any one of claims 1 to 10, **characterised in that** further steps for purifying the material are carried out, in particular a chromatographic purification.

12. A method according to any one of claims 1 to 11, **characterised in that** a further step for inactivating or depleting, respectively, pathogens is carried out, in particular a filtration or a heat treatment.

13. A method according to any one of claims 1 to 12, **characterised in that** a non-ionic detergent selected from the group of Tween and Triton is used as the chemical agent.

14. A chromatographically purified preparation, comprising an autodynamically activatable blood factor with a portion of activated blood factor of less than 50%, based on the content of activated and non-activated blood factor, preferably less than 40%, more preferred less than 30%, still more preferred less than 20%, further preferred less than 10%, most preferred less than 1%, and a detergent content.

15. A preparation according to claim 14, **characterised in that** the blood factor is selected from the group of factor VII, factor XII, factor XI and pre-kallikrein.

16. A preparation according to any one of claims 14 or 15, **characterised in that** it contains a prothrombin complex with a factor VIIa activity of less than 50%, based on the content of activated and non-activated factor VII, preferably less than 10%, most preferred less than 1%.

17. A preparation according to any one of claims 14 to 15, **characterised in that** the preparation is free from serine protease inhibitors and the cofactors thereof, respectively.

18. A preparation according to any one of claims 14 to 17, obtainable by a method according to any one of claims 1 to 13.

## Revendications

1. Procédé d'inactivation d'agents pathogènes, en particulier de virus dans un matériau biologique par incubation dans un milieu chimique, **caractérisé en ce que** le matériau biologique est adsorbé sur un support solide puis **en ce que** l'incubation avec un milieu chimique est réalisée en présence d'un sel éluotrope, correspondant à une concentration de NaCl d'au moins 200 mM, de préférence d'au moins 300 mM, pour la dissolution et/ou la séparation de la substance adsorbée du support, l'incubation se déroulant en même temps que l'élution du matériau biologique ou directement après.

2. Procédé selon la revendication 1, **caractérisé en ce que** le milieu chimique utilisé est un détersif, qui est présent de préférence dans une quantité d'au moins 1%, plus préférentiellement de plus de 5%, de façon préférée entre toutes de plus de 10%.

3. Procédé selon la revendication 1, **caractérisé en ce que** le sel éluotrope chlorure de sodium est utilisé.

4. Procédé selon la revendication 1 à 3, **caractérisé en ce que** l'incubation a lieu pendant une période de 10 minutes à 10 heures, de façon préférée entre toutes de 1 heure à 5 heures.

5. Procédé selon la revendication 1 à 4, **caractérisé en ce que** du plasma ou une fraction de plasma est utilisée comme matériau biologique, ou bien un matériau provenant de culture cellulaire.

6. Procédé selon la revendication 1 à 5, **caractérisé en ce qu'**un matériau biologique est utilisé qui contient un facteur sanguin, en particulier une protéine dépendante de la vitamine K.

7. Procédé selon la revendication 1 à 6, **caractérisé en ce qu'**on utilise un matériau biologique qui est une fraction contenant le complexe prothrombique.

8. Procédé selon la revendication 1 à 7, **caractérisé** en ce le matériau biologique est adsorbé sur un support solide, est purifié et en ce que l'incubation est réalisée après élution du matériau purifié.

9. Procédé selon la revendication 8, **caractérisé en ce qu'**un matériau chromatographique est utilisé comme support solide, en particulier un matériau adapté pour la chromatographie par échange d'ions ou la chromatographie par affinité.

10. Procédé selon la revendication 8 ou 9, **caractérisé en ce qu'**un matériau chromatographique est utilisé comme support solide, en particulier un matériau adapté pour la chromatographie par échange d'ions ou la chromatographie par affinité.

11. Procédé selon la revendication 1 à 10, **caractérisé en ce que** d'autres étapes de purification du matériau sont réalisées, en particulier une purification chromatographique.

12. Procédé selon la revendication 1 à 11, **caractérisé en ce qu'**une autre étape d'inactivation ou d'affaiblissement d'agents pathogènes est réalisée, en particulier une filtration ou un traitement thermique.

13. Procédé selon la revendication 1 à 12, **caractérisé en ce qu'**on utilise comme agent chimique un détersif non-ionique provenant du groupe Tween ou Triton.

14. Préparation purifiée par chromatographie, contenant un facteur sanguin activable de façon autodynamique avec une part de facteur sanguin activé inférieure à 50% par rapport à la teneur en facteur sanguin activé et non activé, de préférence inférieure à 40%, plus préférablement inférieure à 30%, plus préférablement encore inférieure à 20%, encore plus préférablement inférieure à 10%, de façon préférée entre toutes inférieure à 1%, et une part de détersif.

15. Préparation selon la revendication 14, **caractérisée en ce que** le facteur sanguin est sélectionné dans le groupe de facteur VII, de facteur XI et la pré-kallicréine.

16. Préparation selon la revendication 14 ou 15, **caractérisé en ce qu'**elle contient un complexe prothrombique avec un facteur d'activité VIIa inférieur à 50%, comparé à la teneur en facteur VII activé et non activé, de préférence inférieur à 10%, de façon préférée entre toutes inférieur à 1%.

17. Préparation selon la revendication 14 à 15, **caractérisée en ce que** la préparation est vierge d'inhibiteurs de protéase sérique ou de leurs co-facteurs.

18. Préparation selon la revendication 14 à 17, que l'on peut obtenir par un procédé selon l'une des revendications 1 à 13.
